# EUROPEAN PATENT APPLICATION

(11) **EP 2 478 887 A1**
(43) Date of publication of application: **25.07.2012**
(21) Application number: 10816871.7
(22) Date of filing: 14.09.2010
(51) Int. Cl.: A61J 3/00, G06Q 50/00

(54) **METHOD FOR CONTROLLING DRUG MANAGEMENT APPARATUS AND DRUG MANAGEMENT APPARATUS**

(30) Priority: 17.09.2009 JP 2009215340
(71) Applicant: Panasonic Corporation, Kadoma-shi Osaka 571-8501 (JP)
(72) Inventor: TANIMOTO, Takanobu, Chuo-ku, Osaka-shi Osaka 540-6207 (JP); GOTOU, Makoto, Chuo-ku, Osaka-shi Osaka 540-6207 (JP); MATSUKAWA, Yoshihiko, Chuo-ku, Osaka-shi Osaka 540-6207 (JP); KUWATA, Hidenori, Chuo-ku, Osaka-shi Osaka 540-6207 (JP); KENJOU, Noriko, Chuo-ku, Osaka-shi Osaka 540-6207 (JP)
(74) Representative: Vigand, Philippe
(86) International application number: PCT/JP2010/005591
(87) International publication number: WO 2011/033756

(57) **Abstract**

A medicine management apparatus includes: a medicine storage (11); a tag detection unit (14) which detects (i) worker information for identifying a worker and a work position of the worker and (ii) worker information and a work position of a worker who is present in the first work area and worker information and a work position of a worker who is present in the second work area; a management unit (22) which determines, based on a result of the detection performed by the tag detection unit (14), whether or not an unlocking condition is satisfied, which is (i) plural workers who are authorized to use the medicine storage (11) are present in an area that is a combination of the first work area and the second work area and (ii) at least one of the workers is present in the first work area, in the state where the medicine storage (11) is locked, and a lock unit (13) which sets the medicine storage (11) to a state which allows the medicine storage to be unlocked, when it is determined, by the management unit (22), that the unlocking condition is satisfied.

## Description

### [Technical Field]

The present invention relates to a medicine management apparatus which stores medicines to be dispensed in a hospital or a pharmacy.

### [Background Art]

In a hospital, a doctor diagnoses patients suffering from a variety of disorders or injuries, and prepares prescription information for treatment. Medicines are dispensed according to the prescription information in a pharmacy or a prescription department.

In general, the dispensing of medicines is manually carried out by a worker. Although an automatic medicine dispenser can be used for the dispensing of medicines, certain medicines which require an especially careful management are dispensed manually by a worker.

Since the dispensing of medicines performed by a worker is a human work, there is a possibility of an error in the dispensing.

In order to prevent an error in dispensing, there is a medicine storing device having a container of a medicine that is described in prescription information, which opens when the prescription information is input (see, for example, PTL 1).

As shown in FIG. 1, a medicine storing device 1 described in PTL 1 opens a door 3 or a drawer 4 upon reading a prescription document by a bar-code reader 2 and causes a display 5 or 6 which is provided to each of the containers and indicates a number to display the number of medicines to be taken out. With this, the location where the desired medicine is stored is obvious at a glance and at the same time the number of medicines to be taken out is indicated right in front.

As described above, the medicine storing device 1 assists dispensing work, so that the dispensing work is facilitated, and thus it is possible to reduce errors. However, since the dispensing work is a human work, there remains the possibility of an error in the dispensing.

In addition, some pharmacies or prescription departments set their own rules (double-check system), in order to prevent an error in dispensing, such that the dispensing work of medicines is performed by two people including a worker who performs the dispensing of medicines (dispenser) and a worker who checks the work performed by the dispenser (checker).

According to the double-check system with which the dispensing of medicines is performed by the dispenser and the checker, the dispenser and the checker place their seals or signatures on a document on which prescription information is recorded, thereby ensuring that double check is carried out.

### [Citation List]

### [Patent Literature]

### [PTL 1]

Japanese Unexamined Patent Application Publication No. 2004-187958

### [Summary of Invention]

### [Technical Problem]

The problem of conventional medicine management apparatuses lies in that medicines are approved by a human double-check system.

Even when there are seals or signatures of the dispenser and the checker on the document, since placing a seal or a signature is a human work, there is a possibility that the seal of the checker is placed by the dispenser.

More specifically, when a seal of the worker is entrusted to the dispenser, the seal of the dispenser and the checker can be placed on the document without the presence of the checker during the work of the dispenser. In addition, it is also likely that the checker places his seal or signature at a time without confirming the details of dispensing of medicines after the dispensing work is completed.

The present invention solves the problems of the conventional medicine management apparatuses and has an object to provide a medicine management apparatus and a method of controlling the medicine management apparatus for enabling double check to be carried out reliably at the time of dispensing of medicines.

### [Solution to Problem]

In order to achieve this object, the method of controlling a medicine management apparatus that includes a medicine storage and a detection unit that detects worker information for identifying a worker and a work position of the worker, wherein a first work area and a second work area are preliminarily specified, the first work area including an installation position of the medicine management apparatus, and the second work area including a position from which the worker who operates the medicine management apparatus is directly visible, comprises: detecting the worker information and the work position of the worker; determining whether or not an unlocking condition is satisfied, based on a result of the detecting, the unlocking condition being that, in a state where the medicine storage is locked, (i) a plurality of workers authorized to use the medicine storage are present in an area that is a combination of the first work area and the second work area and (ii) at least one of the workers is present in the first work area; and setting the medicine storage to a state which allows the medicine storage to be unlocked, when it is determined, in the determining whether or not an unlocking condition is satisfied, that the unlocking condition is satisfied.

According to the above-described structure, the medicine storage cannot be unlocked unless two workers who are authorized to use the medicine storage are present near the medicine management apparatus. It is therefore possible to reliably perform double check at the time of dispensing of medicines.

Preferably, the method of controlling a medicine management apparatus described above further comprises: determining whether or not a working condition is satisfied, based on the result of the detecting, the working condition being that, in a state where the medicine storage is unlocked, (i) the plurality of workers authorized to use the medicine storage are present in the area that is the combination of the first work area and the second work area and (ii) the at least one of the workers is present in the first work area; and generating an alert signal when it is determined, in the determining whether or not a working condition is satisfied, that the working condition is not satisfied.

According to the above-described structure, an alert is issued when one of the workers disappears from the vicinity of the medicine management apparatus during the dispensing of medicines. This allows preventing each of the workers from being left alone near the medicine management apparatus. It is therefore possible to reliably perform double check at the time of dispensing of medicines.

More preferably, the method of controlling a medicine management apparatus described above further comprises locking the medicine storage when it is determined, in the determining whether or not a working condition is satisfied, that the working condition is not satisfied.

According to the above-described structure, the medicine storage is locked when one of the workers disappears from the vicinity of the medicine management apparatus during the dispensing of medicines. This allows preventing each of the workers from performing the dispensing of medicines when being left alone near the medicine management apparatus.

In addition, in the detecting, the worker information and the work position of the worker may be detected by analyzing a captured image of the worker.

According to the above-described structure, it is possible to prevent impersonation using a name tag (an ID tag) of others, by using the captured image of the worker.

Preferably, in the determining whether or not an unlocking condition is satisfied, whether or not the unlocking condition is satisfied is determined based on a result of the detection in the detecting, the unlocking condition being that, in the state where the medicine storage is locked, (i) at least one of the workers authorized to work in the first work area is present in the first work area and (ii) at least one of the workers authorized to work in the second work area is present in the second work area.

According to the above-described structure, it is possible to determine whether or not the unlocking condition is satisfied with clear distinction between the worker who is authorized to work in the first work area and the worker who is authorized to work in the second work area.

In addition, in the determining whether or not a working condition is satisfied, whether or not the working condition is satisfied may be determined, based on a result of the detection in the detecting, the working condition being that, in the state where the medicine storage is unlocked, (i) at least one of the workers authorized to work in the first work area is present in the first work area and (ii) at least one of the workers authorized to work in the second work area is present in the second work area.

According to the above-described structure, it is possible to determine whether or not the working condition is satisfied with clear distinction between the worker who is authorized to work in the first work area and the worker who is authorized to work in the second work area.

Preferably, a checking appratus for receiving a result of check performed by a worker is located in the second work area, and the method further comprises: determining whether or not a check condition is satisfied, the check condition being that at least one checker who is a worker authorized to check medicines is present in the second work area; and receiving a result of check performed by the checker when it is determined, in the determining whether or not a check condition is satisfied, that the check condition is satisfied.

According to the above-described structure, the medicine management apparatus can receive a check result only when the worker who is authorized to check medicines is present in the second work area. It is therefore possible to prevent an unauthorized check.

In addition, the checking appratus may be located in the second work area and outside the first work area, and in the determining whether or not a check condition is satisfied, it may be determined whether or not a check condition that the at least one checker is present in the second work area and outside the first work area is satisfied.

In addition, the first work area and the second work area may be preliminarily specified such that a worker cannot operate the checking appratus from the first work area and a worker cannot operate the medicine management apparatus from the second work area.

In addition, an area which belongs to none of the work areas may be provided between the first work area and the second work area.

Since the position of the medicine management apparatus and the position of the checking appratus can be clearly separated, it is possible to accurately identify the worker who operates the checking apparatus, and thus it is possible to prevent unauthorized checking from being performed by the worker who is in the first work area.

In addition, the medicine management apparatus may further include a check result receiving unit for receiving a result of check, and the method may further comprise: determining whether or not a check condition is satisfied, the check condition being that at least one checker who is a worker authorized to check medicines is present in the first work area; and receiving a result of check performed by the checker when it is determined, in the determining whether or not a check condition is satisfied, that the check condition is satisfied.

According to the above-described structure, the medicine management apparatus can receive a check result only when the medicine management apparatus is provided with a function to perform the checking and the worker who is authorized to check the medicines is present in the first work area. It is therefore possible to prevent unauthorized checking performed by a person other than the checker.

Preferably, in the determining whether or not a check condition is satisfied, it may be determined whether or not a check condition that the at least one checker is present in the first work area and a worker unauthorized to check medicines is not present in the first work area is satisfied.

According to the above-described structure, it is ensured that only the checker is present in the first work area. It is therefore possible to further reliably prevent unauthorized checking performed by a person other than the checker.

It is to be noted that the present invention can be implemented not only as a method of controlling the medicine management apparatus which includes characteristic steps described above but also as a medicine management apparatus including, as processing units, the characteristic steps included in the method of controlling the medicine management apparatus or as a program for causing a computer to execute the characteristic steps included in the method of controlling the medicine management apparatus. It should be understood that such a program can be distributed via a non-transitory computer-readable recording medium such as a CD-ROM (Compact Disc-Read Only Memory) or the like and a communication network such as the Internet.

### [Advantageous Effects of Invention]

According to the present invention, since medicine dispensing work cannot be carried out unless there are two workers, it is possible to provide a method of controlling a medicine management apparatus and an appratus thereof which enable double check to be performed reliably at the time of dispensing of medicines.

### [Brief Description of Drawings]

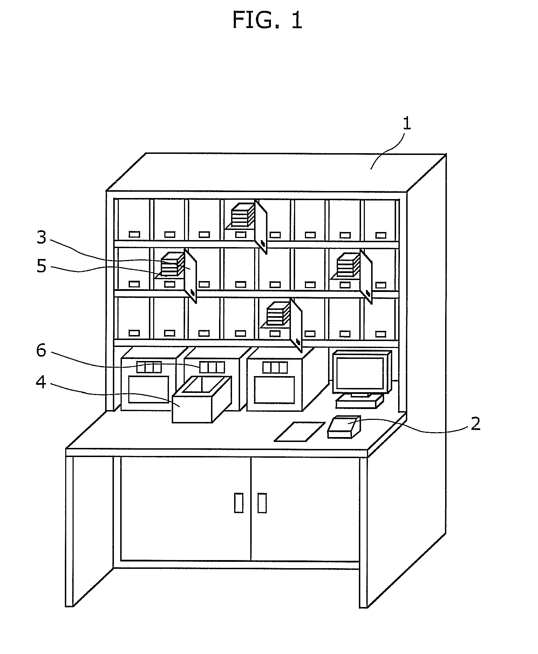

[FIG. 1] FIG. 1 is a diagram which shows a perspective view of a conventional medicine storing device.
[FIG. 2] FIG. 2 is a diagram which shows a perspective view of a medicine management apparatus in an open state according to Embodiment 1 of the present invention.
[FIG. 3] FIG. 3 is a diagram which shows a perspective view of the medicine management apparatus in a closed state according to Embodiment 1 of the present invention.
[FIG. 4] FIG. 4 is a diagram which shows a front view of a name tag according to Embodiment 1.
[FIG. 5] FIG. 5 is a block diagram which shows a functional structure of the medicine management apparatus according to Embodiment 1.
[FIG. 6] FIG. 6 is a diagram which shows an example of worker information of a worker who is authorized to use a medicine storage, which is stored in a user registration unit.
[FIG. 7] FIG. 7 is a diagram which shows an external view of a checking appratus.
[FIG. 8] FIG. 8 is a block diagram which shows a functional structure of the checking appratus.
[FIG. 9] FIG. 9 is a diagram which shows a detection area of the medicine management apparatus according to Embodiment 1.
[FIG. 10] FIG. 10 is a diagram which shows another detection area of the medicine management apparatus.
[FIG. 11] FIG. 11 is a flow chart of a determination process for an unlocking condition, which is performed at the time of starting dispensing of medicines according to Embodiment 1.
[FIG. 12] FIG. 12 is a diagram which shows an example of the location of workers when the unlocking condition is satisfied.
[FIG. 13] FIG. 13 is a diagram which shows an example of the location of workers when the unlocking condition is satisfied.
[FIG. 14] FIG. 14 is a diagram which shows an example of the location of workers when the unlocking condition is not satisfied.
[FIG. 15] FIG. 15 is a diagram which shows an example of the location of workers when the unlocking condition is not satisfied.
[FIG. 16] FIG. 16 is a flow chart of a determination process for a working condition, which is performed during the dispensing of medicines according to Embodiment 1.
[FIG. 17] FIG. 17 is a flow chart of a determination process for a check condition, which is performed during check of medicines according to Embodiment 1.
[FIG. 18] FIG. 18 is a diagram which shows a perspective view of a medicine management apparatus according to Embodiment 2 of the present invention.
[FIG. 19] FIG. 19 is a block diagram which shows a functional structure of a medicine management apparatus according to Embodiment 3
[FIG. 20] FIG. 20 is a diagram which shows an example of worker information of a worker who is authorized to use a medicine storage, which is registered in a first authorized user registration unit.
[FIG. 21] FIG. 21 is a diagram which shows an example of worker information of a worker who is authorized to use a medicine storage, which is registered in a second authorized user registration unit.
[FIG. 22] FIG. 22 is a flow chart of a determination process for an unlocking condition, which is performed at the time of starting dispensing of medicines according to Embodiment 3.
[FIG. 23] FIG. 23 is a flow chart of a determination process for a working condition, which is performed during the dispensing of medicines according to Embodiment 3.
[FIG. 24] FIG. 24 is a flow chart of a determination process for a check condition, which is performed during check of medicines according to Embodiment 3.
[FIG. 25] FIG. 25 is a diagram which shows an example of the location of workers when the check condition of medicines is satisfied.
[FIG. 26] FIG. 26 is a diagram which shows an example of the location of workers when the check condition of medicines is not satisfied.
[FIG. 27] FIG. 27 is a block diagram which shows a functional structure of a medicine management apparatus according to Modification 1 of Embodiment 1 of the present invention.
[FIG. 28] FIG. 28 is a diagram which shows an example of the location of workers when the unlocking condition is satisfied.
[FIG. 29] FIG. 29 is a diagram which shows an example of the location of workers when the unlocking condition is satisfied.
[FIG. 30] FIG. 30 is a diagram which shows an example of the location of workers when the unlocking condition is not satisfied.
[FIG. 31] FIG. 31 is a diagram which shows an example of the location of workers when the unlocking condition is not satisfied.
[FIG. 32] FIG. 32 is a block diagram which shows a functional structure of a medicine management apparatus according to Modification 2 of Embodiment 3 of the present invention.
[FIG. 33] FIG. 33 is a diagram which shows an example of the location of workers when the check condition of medicines is satisfied.
[FIG. 34] FIG. 34 is a diagram which shows an example of the location of workers when the check condition of medicines is not satisfied.

### [Description of Embodiments]

The following describes embodiments for implementing the present invention, with reference to the drawings. It is to be noted that, in the following description, the same numerals are assigned in the same structure and the explanation is omitted accordingly.

### (Embodiment 1)

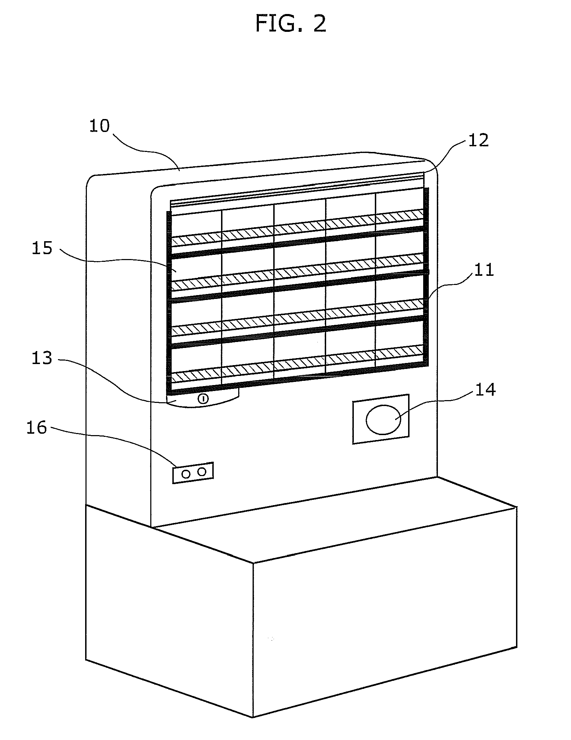

FIG. 2 is a diagram which shows a perspective view of a medicine management apparatus 10 in an open state according to Embodiment 1 of the present invention. Here, the open state is a state where a shutter is open. The medicine management apparatus 10 includes: a medicine storage 11; a lock unit 13; a tag detection unit 14; and an operation unit 16.

The medicine storage 11 is a storage in which a medicine is contained and stored. The lock unit 13 is for unlocking or locking a shutter 12 of the medicine storage 11. The tag detection unit 14 detects worker information and a work position simultaneously in a non-contact manner, from plural identification (ID) tags embedded in plural name tags on the plural workers. The operation unit 16 receives operation, by a worker, for the medicine storage 11.

The medicine storage 11 includes plural containers 15 in which medicines are contained and the shutter 12 that covers the containers 15 at the front surface of the containers 15.

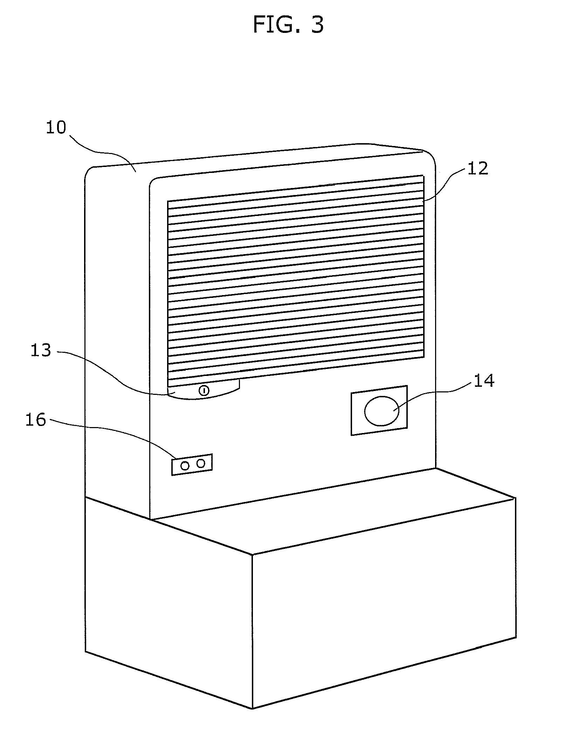

FIG. 3 is a diagram which shows a perspective view of the medicine management apparatus 10 in a closed state according to Embodiment 1. Here, the closed state is a state where a shutter 12 is closed. FIG. 3 shows the medicine management apparatus 10 of which the shutter 12 is closed and locked by the lock unit 13. Thus, in the state shown in FIG. 3, a worker cannot take out a medicine from the medicine storage 11 (container 15).

The tag detection unit 14 is a noncontact ID tag reader. The tag detection unit 14 reads an ID tag embedding in a name tag of a worker and detects worker information of the worker from the result of the reading. In addition, the tag detection unit 14 detects a position where the ID tag is present.

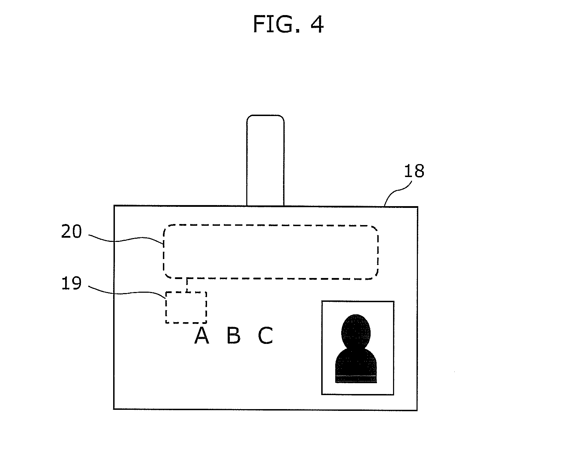

The ID tag embedded in the name tag includes an integrated circuit (IC) 19 and an antenna 20 which are embedding in a name tag 18 of a worker as shown in FIG. 4. Identification information and the like of the worker is recorded on the IC 19 of the ID tag. A radio frequency identification (RFID) tag of an ultra high frequency (UHF) band is used for the ID tag and the ID tag can be detected by the tag detection unit 14 within an access distance of three meters, for example.

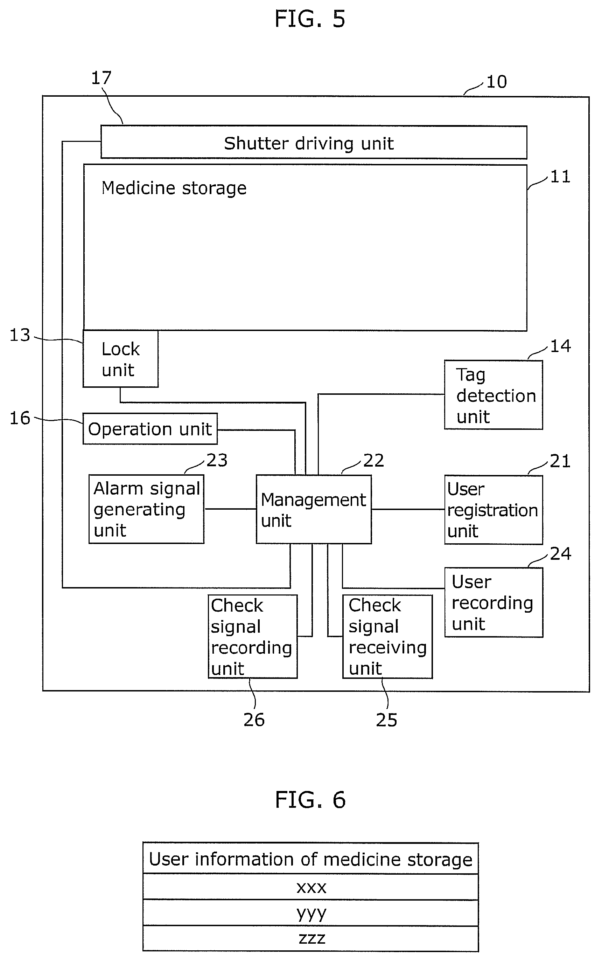

FIG. 5 is a block diagram which shows a functional structure of the medicine management apparatus 10 according to Embodiment 1.

In FIG. 5, the medicine management apparatus 10 includes a shutter driving unit 17, a user registration unit 21, a management unit 22, an alarm signal generating unit 23, a user recording unit 24, a check signal receiving unit 25, and a check signal recording unit 26, in addition to the medicine storage 11, the lock unit 13, the tag detection unit 14, and the operation unit 16.

The shutter driving unit 17 opens and closes the shutter 12 according to movements of the lock unit 13. More specifically, the shutter driving unit 17 opens the shutter 12 when the lock unit 13 unlocks the shutter 12. In addition, the shutter driving unit 17 closes the shutter 12 when the lock unit 13 locks the shutter 12.

The user registration unit 21 stores a worker number that is worker information of a worker authorized to use the medicine storage 11. FIG. 6 is a diagram which shows an example of the worker information of the worker who is authorized to use the medicine storage 11, which is stored in the user registration unit 21. As shown in FIG. 6, "xxx", "yyy", and "zzz" are recorded as the worker numbers of workers authorized to use the medicine storage 11.

The management unit 22 determines whether or not the worker number indicated by the worker information detected by the ID tag is recorded on the user registration unit 21.

The alarm signal generating unit 23 generates an alarm signal.

The worker number is a number for identifying a worker included in the worker information detected by the tag detection unit 14.

The user recording unit 24 records the worker number of a worker who is carrying out the medicine dispensing work.

The check signal receiving unit 25 receives a check signal that indicates a result of checking, from a checking appratus 50 described later.

The check signal recording unit 26 records the check signal received by the check signal receiving unit 25.

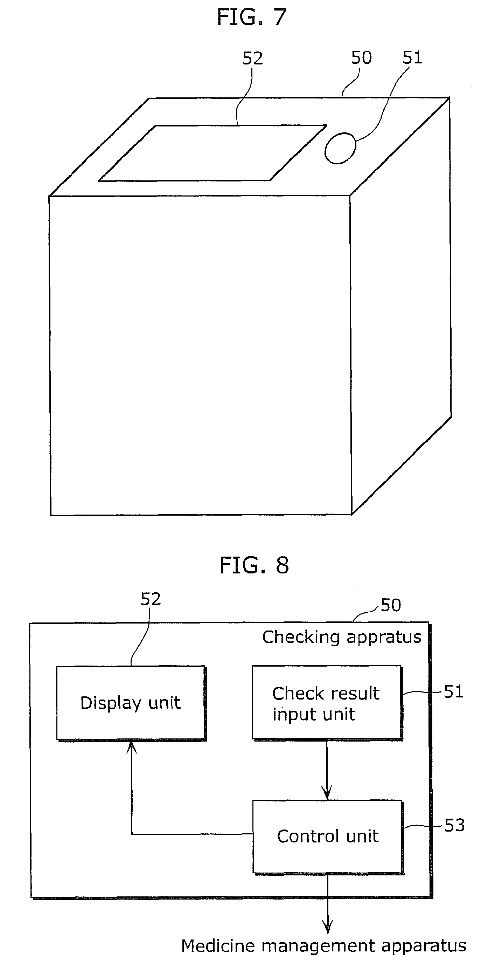

FIG. 7 is a diagram which shows an external view of the checking appratus 50. The checking appratus 50 is an apparatus for inputting a result of checking the medicines dispensed by a dispenser to see whether or not there is an error in the dispensing of the medicines. The checking appratus 50 includes a check result input unit 51 and a display unit 52. The check result input unit 51 is an interface used by a checker to input a result of checking, and formed as a button or the like. The display unit 52 is a display for displaying the result of checking.

FIG. 8 is a block diagram which shows a functional structure of the checking appratus 50. The checking appratus 50 includes the check result input unit 51, the display unit 52, and a control unit 53.

The control unit 53 causes the result of checking received by the check result input unit 51 to be displayed on the display unit 52 and at the same time transmits the result of checking to the medicine management apparatus 10.

Next, a brief description will be given as to a process performed by the medicine management apparatus 10 at the time of starting the medicine dispensing work and during the medicine dispensing work.

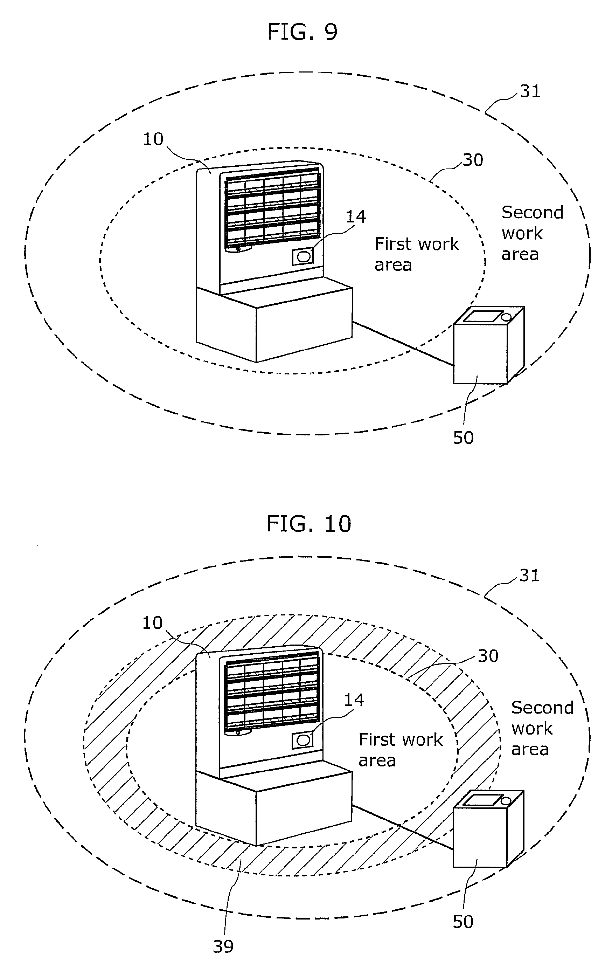

As shown in FIG. 9, a first work area 30 and a second work area 31 are specified around the medicine management apparatus 10. The first work area 30 is an area including an installation position of the medicine management apparatus 10. The second work area 31 is an area including a position from which a worker (a first worker) who operates the medicine management apparatus 10 is directly visible from a worker (a second worker) different from the first worker. The checking appratus 50 is connected to the medicine management apparatus 10 and provided in the second work area 31.

For example, the first work area 30 and the second work area 31 are areas centering around the tag detection unit 14 of the medicine management apparatus 10, as shown in FIG. 9. The first work area 30 is located in the second work area 31, and the second work area 31 includes the first work area 30; that is, those included in the first work area 30 are always included in the second work area 31 as well. To be specific, the first work area 30 is a circular area with a radius of 1 m and the second work area 31 is a circular area with a radius of 5 m, each of which is centering around the tag detection unit 14.

It is to be noted that the second work area 31 need not necessarily include the first work area 30. Thus, there may be the case where the second work area 31 includes only part of the first work area 30 or where the first work area 30 is not included in the second work area 31. At this time, the first work area 30 and the second work area 31 may be specified such that the checking appratus 50 cannot be operated from the first work area 30 and the medicine management apparatus 10 cannot be operated from the second work area 31. In addition, as shown in FIG. 10, the first work area 30 and the second work area 31 which have no overlapping area may be provided, and a buffer area 39 may be provided between the first work area 30 and the second work area 31. The buffer area 39 is an area that does not belong to the first work area 30 and the second work area 31. By providing the buffer area 39, it is possible to prevent (i) operating the checking appratus 50 from the first work area 30 and (ii) operating the medicine management apparatus 10 from the second work area 31.

It is preferable that the ranges of the first work area 30 and the second work area 31 are specified arbitrarily according to own rules and the like of the respective hospitals in which the medicine management apparatus 10 of Embodiment 1 is provided.

The medicine management apparatus 10 determines, at the time of starting medicine dispensing work, whether or not an unlocking condition that at least one worker who is authorized to use the medicine storage 11 is present in the first work area 30 and plural workers who are authorized to use the medicine storage 11 are present in an area that is a combination of the first work area 30 and the second work area 31 is satisfied. When the unlocking condition is satisfied, the lock unit 13 unlocks the shutter 12. Then the worker can perform dispensing of medicines using the medicine storage 11. It is to be noted that the lock unit 13 may set the shutter 12 to a state which allows the shutter 12 to be unlocked and actual unlocking operation may be performed by the worker.

In addition, the medicine management apparatus 10 determines, in the state that the shutter 12 is unlocked, whether or not the working condition that at least one worker who is authorized to use the medicine storage 11 is present in the first work area 30 and plural workers who are authorized to use the medicine storage 11 are present in an area that is a combination of the first work area 30 and the second work area 31 is satisfied. When the working condition is not satisfied, the alarm signal generating unit 23 generates an alarm signal and the lock unit 13 locks the shutter 12 after a predetermined period of time. One example of the case where the alarm signal generating unit 23 generates an alarm signal and the lock unit 13 locks the shutter 12 after a predetermined period of time is, for example, the case where two workers detected at the same time by the tag detection unit 14 are not detected together at the same time in the first work area 30. In addition, another example is the case where one of the workers is detected in the second work area 31 which includes the first work area 30 and is larger than the first work area 30, while the other worker is not detected in the first work area 30. Thus, the medicine management apparatus 10 according to Embodiment 1 can prevent each of the workers from performing dispensing of medicines when being left alone near the medicine management apparatus 10.

According to the above-described structure, the medicine storage 11 does not open and the shutter 12 is not unlocked unless two workers who are authorized to use the medicine storage 11 are present near the medicine management apparatus 10. In addition, when one of the workers moves away from the medicine management apparatus 10 during the dispensing work, the alarm signal generating unit 23 generates an alarm signal. The alarm signal causes (i) the shutter 12 to be closed or (ii) an alarm sound generating unit to generate an alarm sound. This allows preventing each of the workers from being left alone near the medicine management apparatus 10.

Thus, two workers are inevitably required for performing dispensing of medicines using the medicine management apparatus 10 of Embodiment 1, and thus double check is reliably carried out by two workers.

Next, an operation at the time of starting the dispensing of medicines using the medicine management apparatus 10 will be described in detail.

When the dispensing work is not performed, the shutter 12 of the medicine management apparatus 10 is closed as shown in FIG. 3 and the lock unit 13 locks the shutter 12.

In order to perform dispensing of medicines, it is necessary to open the shutter 12 such that medicines can be taken out from the container 15.

The lock unit 13 unlocks the shutter 12 when the unlocking condition is satisfied. The unlocking condition is that at least one worker who is authorized to use the medicine storage 11 (a worker whose worker number is registered in the user registration unit 21) is present in the first work area 30 and plural workers who are authorized to use the medicine storage 11 are present in an area that is a combination of the first work area 30 and the second work area 31. Then, the shutter driving unit 17 (shown in FIG. 5) contained in the upper portion of the medicine management apparatus 10 opens the shutter 12 according to movement of the lock unit 13. Then, as shown in FIG. 2, the shutter 12 of the medicine management apparatus 10 opens, making it possible to open the container 15 of the medicine storage 11.

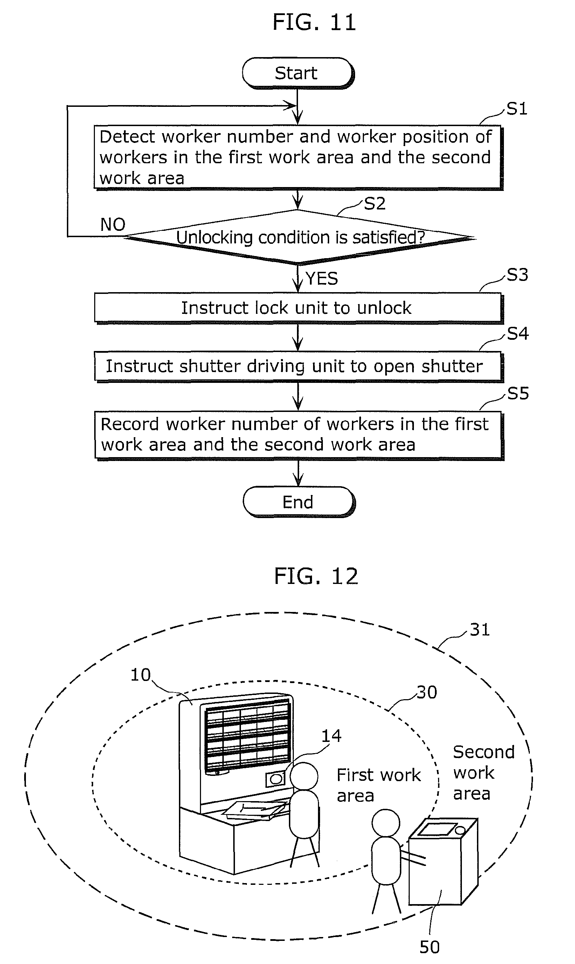

The following describes a determination process for an unlocking condition carried out at the time of starting dispensing of medicines, with reference to the flow chart shown in FIG. 11.

The tag detection unit 14 detects an ID tag embedded in the name tag 18 of a worker who is present at a detectable position, and transmits, to the management unit 22, a worker number recorded on the ID tag and a detection position of the detected ID tag (S1). At this time, it is possible to simultaneously detect plural ID tags. However, the case where ID tags of two workers are detected is described here.

The management unit 22 determines whether or not the worker numbers and the work positions of the two workers transmitted from the tag detection unit 14 satisfy the unlocking condition for unlocking the shutter 12 by the lock unit 13 (S2).

The unlocking condition, here, means that the following two items are simultaneously satisfied.
[Unlocking condition 1] Each of the detected worker numbers of plural workers are registered on the user registration unit 21.
[Unlocking condition 2] The detected work positions of plural workers are in the area that is a combination of the first work area 30 and the second work area 31, and the work position of one of the workers is in the first work area 30.

Here, an example of the location of workers when the unlocking condition is not satisfied will be explained.

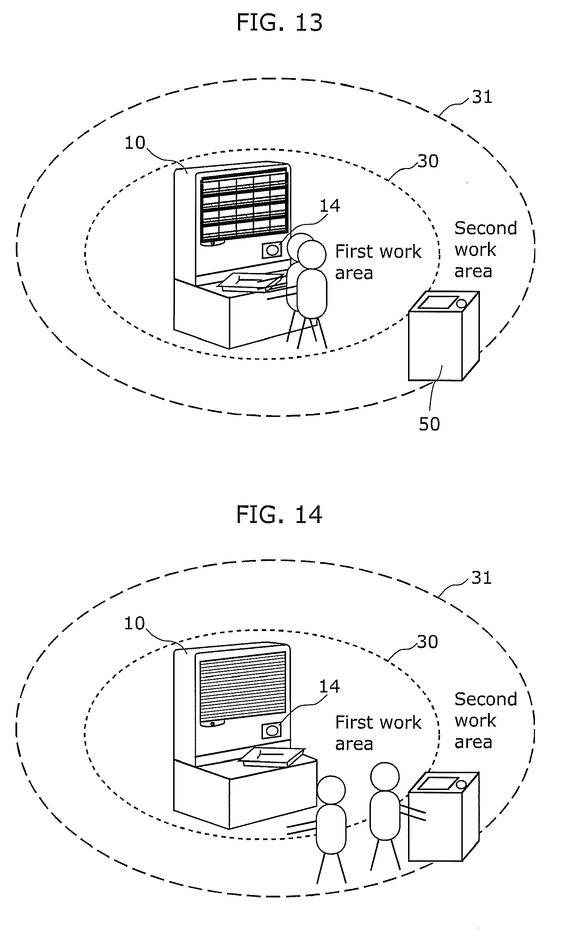

FIG. 12 and FIG. 13 show examples of the location of the workers when the unlocking condition is satisfied.

As shown in FIG. 12, when the worker whose worker number is registered on the user registration unit 21 is present in the first work area 30 and the worker whose worker number is registered on the user registration unit 21 is present in the second work area 31, it is determined that the unlocking condition is satisfied.

In addition, as shown in FIG. 13, when two workers whose worker numbers are registered on the user registration unit 21 are present in the first work area 30, it is determined that the unlocking condition is satisfied as well.

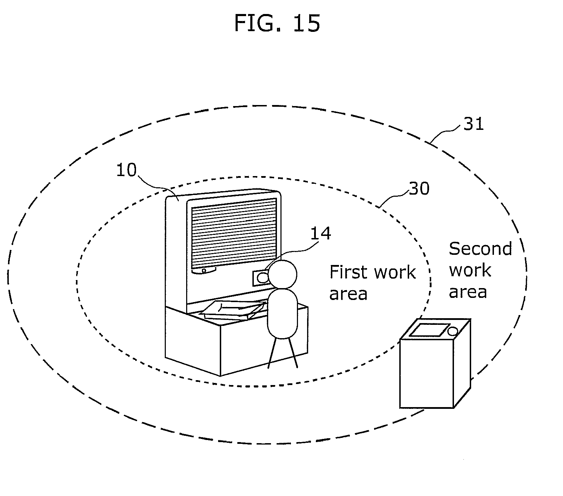

FIG. 14 and FIG. 15 show examples of the location of the workers when the unlocking condition is not satisfied.

As shown in FIG. 14, when two workers whose worker numbers are registered on the user registration unit 21 are present in the second work area 31 and outside the first work area 30, it is determined that the unlocking condition is not satisfied. More specifically, it is required, by the above-described Unlocking condition 2, that a worker is present in the first work area 30, and thus it is determined that the unlocking condition is not satisfied because the condition is not satisfied.

In addition, as shown in FIG. 15, when a worker whose worker number is registered on the user registration unit 21 is present in the first work area 30 but there is no worker in the second work area 31, the above-described Unlocking condition 1 and Unlocking condition 2 are not satisfied, and thus it is determined that the unlocking condition is not satisfied.

When the two items are not simultaneously satisfied (NO in S2), it is determined that the unlocking condition is not satisfied and the process returns to S1. Then the management unit 22 repeats the determination of whether or not the worker number and the work position transmitted from the tag detection unit 14 satisfy the unlocking condition, until the above-described two items are simultaneously satisfied.

When the unlocking condition is satisfied (YES in S2), the management unit 22 instructs the lock unit 13 to unlock the shutter 12 (S3). The lock unit 13 unlocks the shutter 12 upon receiving the instruction from the management unit 22.

The management unit 22 instructs the shutter driving unit 17 to open the shutter 12 to make the medicine storage 11 available (S4). The shutter driving unit 17, in response to the instruction from the management unit 22, opens the shutter 12 to make the medicine management apparatus 10 in the state shown in FIG. 3.

The management unit 22 records the detected worker numbers of the two workers on the user recording unit 24 (S5).

As described above, when the shutter 12 is opened and medicines can be taken out from the container 15, it is possible for the worker to dispense medicines.

Next, an operation during the dispensing of medicines using the medicine management apparatus 10 will be described.

According to Embodiment 1, when the medicine management apparatus 10 is used for dispensing, the dispensing of medicines cannot be continued unless two workers are present. More specifically, the dispensing of medicines cannot be performed unless one of the workers is present in the first work area 30 and the other is present in the second work area 31.

Accordingly, when the medicine management apparatus 10 is used for dispensing, one of the workers performs dispensing work and the other is present in the vicinity, and thus it is possible to ensure that double check is carried out always by two workers.

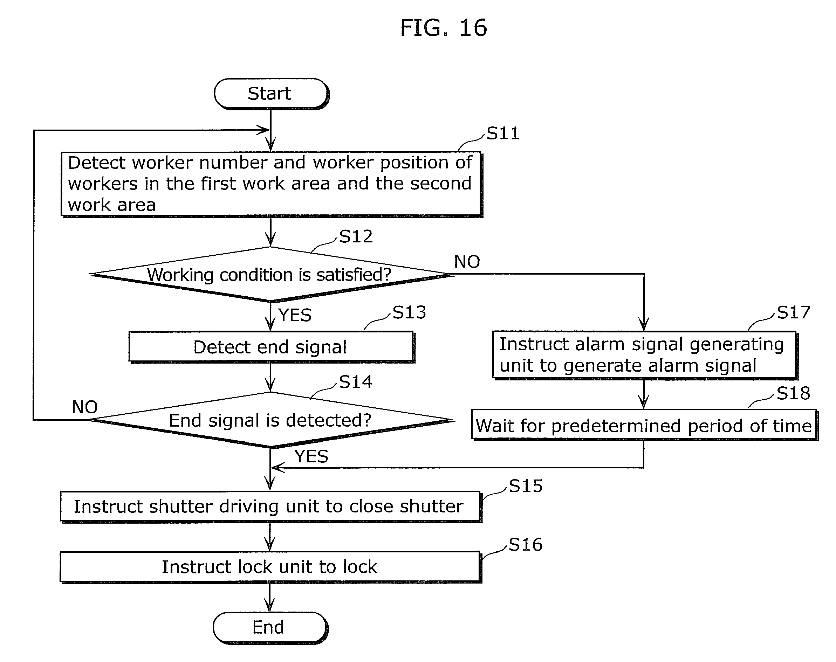

The following describes a determination process for a working condition carried out during dispensing of medicines, with reference to the flow chart shown in FIG. 16.

The tag detection unit 14 detects an ID tag embedded in the name tag 18 of a worker who is present in a detectable distance, and transmits, to the management unit 22, a worker number recorded on the ID tag and a detection position of the detected ID tag (S11).

At this time, it is possible to simultaneously detect plural ID tags. In this case, the ID tags of two workers are detected and the worker numbers and the work positions of the two workers are transmitted to the management unit 22.

The management unit 22 determines whether or not the working condition (a condition for allowing dispensing of medicines) of the medicine management apparatus 10 is satisfied, from the worker numbers and the work positions of the two workers transmitted from the tag detection unit 14 (S12).

The working condition of the medicine management apparatus 10, here, means that the following two items are simultaneously satisfied.
[Working condition 1] Each of the detected worker numbers of plural workers is recorded on the user recording unit 24.
[Unlocking condition 2] Detected work positions of plural workers are in the area that is a combination of the first work area 30 and the second work area 31, and the work position of one of the workers is in the first work area 30.

It is to be noted that, since an example of location of workers when the working condition of the medicine management apparatus 10 is satisfied is the same as the example of location of workers when the Unlocking condition is satisfied shown in FIG. 12 and FIG. 13, the detailed explanation is not repeated. In addition, since an example of location of workers when the working condition of the medicine management apparatus 10 is not satisfied is the same as the example of location of workers when the unlocking condition is not satisfied shown in FIG. 14 and FIG. 15, the detailed explanation is not repeated.

When the working condition is satisfied (YES in S12), the management unit 22 detects an end signal that is generated from the operation unit 16 when the worker presses an end button of the operation unit 16 after the work is completed (S13).

When the end signal of the work is not detected (NO in S14), the process returns to S11 and the management unit 22 repeats the determination of whether or not the working condition of the medicine management apparatus 10 is satisfied.

When the end signal of the work is detected (YES in S14), an instruction to close the shutter 12 is issued to the shutter driving unit 17 (S15). Then the shutter 12 is closed to cover the medicine storage 11. The management unit 22 instructs the lock unit 13 to lock the shutter 12 (S16).

When the working condition is not satisfied (NO in S12), the management unit 22 instructs the alarm signal generating unit 23 to generate an alarm signal (S17). The alarm signal generating unit 23 generates an alarm signal in response to the instruction. The management unit 22 performs the processes subsequent to S15 after a predetermined waiting period of time (S18).

When the working condition is satisfied as described above, the worker can take out medicines stored in the container 15, and thus it is possible to perform dispensing of medicines. In addition, when the work is to be ended, the worker presses an end button, thereby making the medicine management apparatus 10 in a closed state.

It is to be noted that, when an alarm signal is generated by the alarm signal generating unit 23 in response to the instruction from the management unit 22, the alarm signal is transmitted to an alarm sound generating unit (not illustrated) of the medicine management apparatus 10 to generate an alarm sound from the alarm sound generating unit. The generated alarm sound notifies, to the surrounding area of the medicine management apparatus 10, that dispensing of medicines is not carried out by the two workers.

In addition, when an alarm signal is generated by the alarm signal generating unit 23, the shutter driving unit 17 receives the alarm signal and automatically closes the shutter 12 to make the medicine storage 11 unavailable, and further the lock unit 13 locks the shutter 12, thereby prohibiting carrying out the dispensing of medicines.

Next, an operation during check of medicines using the medicine management apparatus 10 and the checking appratus 50 will be described.

A person who checks medicines must be qualified to check medicines. The person who is qualified to check medicines is, for example, a person who is a licensed pharmacist. According to Embodiment 1, a worker whose worker number is registered on the user registration unit 21 is a licensed pharmacist.

In Embodiment 1, when a check condition that at least one checker who is authorized to check medicines is present in the second work area 31 is satisfied, the medicine management apparatus 10 receives a result of check performed by the checking appratus 50. It is to be noted that the check of medicines and the dispensing of medicines are performed in parallel.

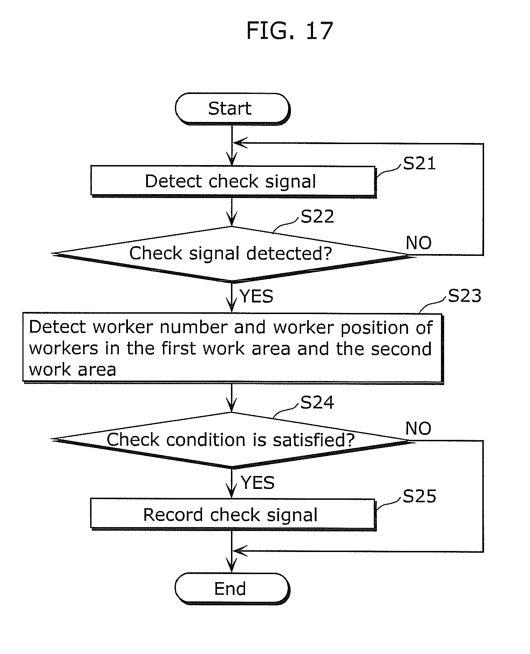

The following describes a determination process for the check condition, which is carried out during the check of medicines, with reference to the flow chart shown in FIG. 17.

In response to an input of a check result by a worker using the check result input unit 51, the control unit 53 transmits a check signal indicating the check result to the check signal receiving unit 25.

The management unit 22 detects whether or not the check signal receiving unit 25 receives the check signal (S21). When the check signal is not detected (NO in S22), the process returns to S21.

When the check signal is detected (YES in S22), The tag detection unit 14 detects an ID tag embedded in the name tag 18 of a worker who is present in a detectable distance, and transmits, to the management unit 22, a worker number recorded on the ID tag and a detection position of the detected ID tag (S23).

At this time, it is possible to simultaneously detect plural ID tags. In this case, the ID tags of two workers are detected and the worker numbers and the work positions of the two workers are transmitted to the management unit 22.

The management unit 22 determines whether or not the check condition of medicines (a condition for allowing check of medicines) is satisfied, from the worker numbers and the work positions of the two workers transmitted from the tag detection unit 14 (S24).

The check condition of medicines, here, means that the following two items are simultaneously satisfied.
[Check condition 1] At least one worker is present in the second work area 31.
[Check condition 2] The worker number of the at least one worker present in the second work area 31 is registered on the user registration unit 21.

When the check condition of medicines is satisfied (YES in S24), the management unit 22 records the check signal received by the check signal receiving unit 25, on the check signal recording unit 26 (S25). With this, the result of check performed by the checker is received.

When the check condition of medicines is not satisfied (NO in S24), the management unit 22 ends the process without recording, on the check signal recording unit 26, the check signal received by the check signal receiving unit 25.

As described above, the medicine management apparatus 10 can receive the check result only when the worker whose worker number is registered on the user registration unit 21 is present in the second work area 31. It is therefore possible to prevent unauthorized checking performed by a person other than the checker.

It is to be noted that the condition may be set as follows: the checking appratus 50 is located in the second work area 31 and outside the first work area 30 and, instead of the above-described check condition 1, at least one worker is present in the second work area 31 and outside the first work area 30. This allows clear separation between the position of the medicine management apparatus 10 and the position of the checking appratus 50, and thus it is possible to accurately identify the worker number of the worker who operates the checking appratus 50. It is therefore possible to prevent unauthorized checking performed by a person other than the checker.

As described above, with the medicine management apparatus 10 according to Embodiment 1, dispensing of medicines cannot be performed unless at least two workers are present in a predetermined area. It is therefore possible to provide a medicine management apparatus with which dispensing of medicines is reliably double checked by at least two workers.

In addition, according the Embodiment 1, it is only necessary for one of the workers who does not perform the dispensing work to be in the first work area 30 or the second work area 31 during the dispensing of medicines. This allows one of the workers who check the dispensing of medicines to carry out the checking in a position not to obstruct the dispensing of medicines performed by the other worker.

It is to be noted that, according to Embodiment 1, the shutter 12 is closed and locked in order to make the medicine storage 11 unavailable (a state unavailable to take out medicines from the container 15). However, the container 15 itself may be locked without using the shutter 12 in order to make the medicine storage 11 unavailable; that is, a state unavailable to take out medicines from the container 15. According to the present invention, the above-described cases are collectively described as "lock the medicine storage 11", including the embodiments below.

In addition, according to Embodiment 1, an alarm sound is issued as an alert when the dispensing of medicines is not performed by two workers. However, the method of alerting the surrounding area is not limited to this. It is possible to alert, for example, by sending information to a monitoring post (not illustrated), by causing a light emitting unit (not illustrated) attached to the medicine management apparatus 10 to emit light, and so on.

### (Embodiment 2)

A medicine management apparatus 33 according to Embodiment 2 of the present invention identifies a worker and detects a work position using an image captured by a camera 32.

The above-described medicine management apparatus 10 according to Embodiment 1 identifies a worker and detects a work position of the worker using an ID tag and the like which is embedded in the name tag 18 of the worker. Therefore, it is possible to impersonate another worker by using a name tag of the other worker to carry out work.

According to Embodiment 2, it is possible to prevent impersonation using a name tag of others, by using an image captured by a camera for identifying a worker.

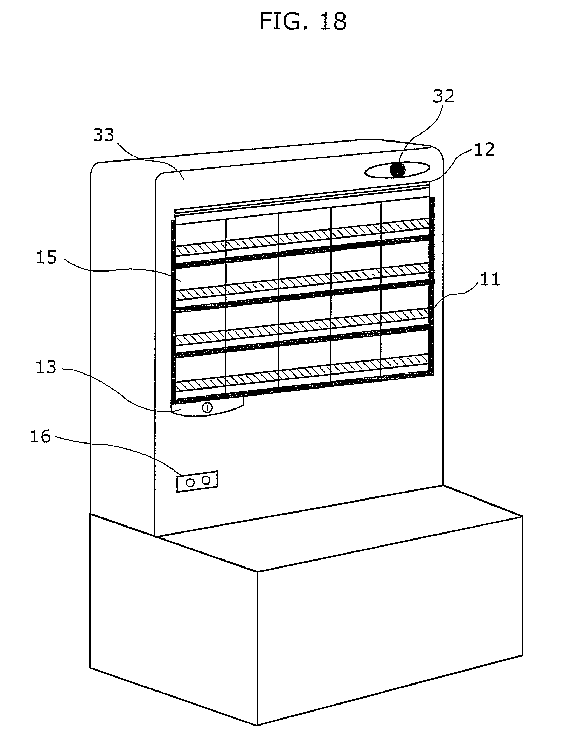

FIG. 18 is a diagram which shows a perspective view of the medicine management apparatus 33 according to Embodiment 2.

The medicine management apparatus 33 includes: the medicine storage 11; the lock unit 13 for unlocking and locking the shutter 12 of the medicine storage 11; and the camera 32 for capturing a worker standing in front of the medicine storage 11.

The medicine management apparatus 33 further include: an image analyzing unit for detecting face images and work positions of two workers using images captured by the camera 32; a user registration unit on which a face image of a worker who is authorized to use the medicine storage is registered; a management unit for determining whether or not the face image detected by the image analyzing unit is registered on the user registration unit; an alarm signal generating unit for generating an alarm signal; a check signal receiving unit for receiving a check signal from the checking appratus; and a check signal recording unit for recording the check signal received by the check signal receiving unit. It is to be noted that conventional techniques can be used for the process of detecting a face image and a work position of a worker from an image, and thus the detailed explanation will be omitted.

The lock unit 13 unlocks the shutter 12 of the medicine storage 11 when an unlocking condition is satisfied. The unlocking condition is that the image analyzing unit simultaneously detects two face images in the state where the shutter 12 of the medicine storage 11 is locked, two workers of the detected two face images are registered together on the user registration unit, the work position of one of the workers is in the first work area 30, and the work position of the other worker is in the first work area 30 or the second work area 31.

In addition, the alarm signal generating unit generates an alarm signal when a working condition of the medicine management apparatus 33 is not satisfied. The working condition is that the image analyzing unit simultaneously detects two face images in the state where the shutter 12 of the medicine storage 11 is unlocked, two workers of the detected two face images are registered together on the user registration unit, the work position of one of the workers is in the first work area 30, and the work position of the other worker is in the first work area 30 or the second work area 31.

Furthermore, the management unit records, on the check signal recording unit, a check signal received by the check signal receiving unit when a check condition is satisfied. The check condition is that the image analyzing unit simultaneously detects two face images, and one worker of one of the detected two face images is registered on the user registration unit and is present in the second work area.

As described above, the medicine management apparatus 33 uses the images captured by the camera, for identifying the workers, thereby preventing impersonation using the name tag (ID tag) of others.

### (Embodiment 3)

A medicine management apparatus 40 according to Embodiment 3 of the present invention separates workers into the dispenser and the checker so as to allow a preparation of medicine such as dispensing and checking when double check by the dispenser and the checker can be performed.

In medical institutions, there is a case where the dispenser and the checker are clearly separated. The medicine management apparatus 40 of Embodiment 3 is a medicine management apparatus that can also be applied to such a case. Here, the checker is a person who is qualified to check medicines, more specifically, a person who is a licensed pharmacist.

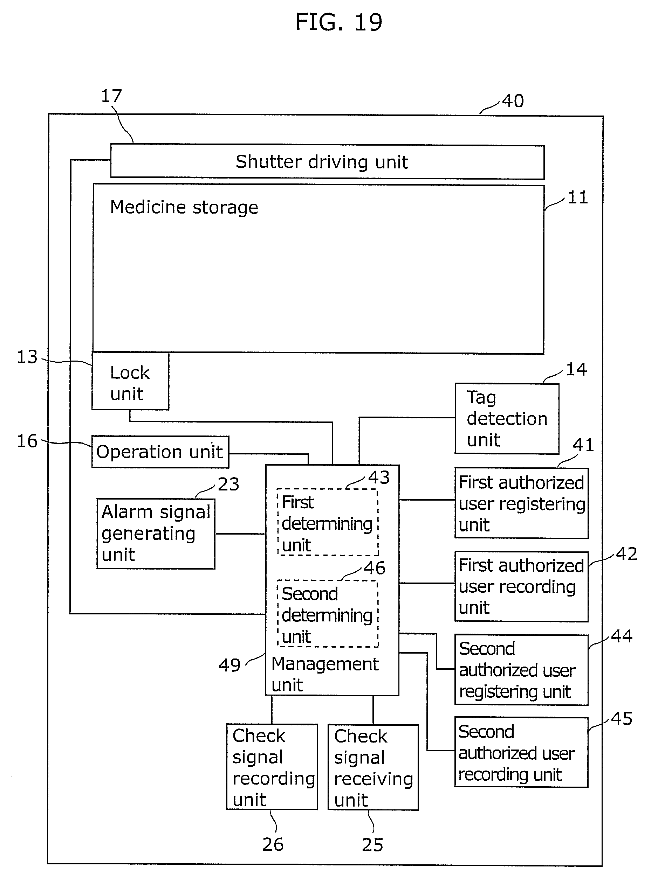

FIG. 19 is a block diagram which shows a functional structure of the medicine management apparatus according to Embodiment 3.

The medicine management apparatus 40 includes: the medicine storage 11; the lock unit 13; and the tag detection unit 14.

The medicine storage 11, the lock unit 13, and the tag detection unit 14 have the same structures as the structures described in the above-described Embodiment 1, and thus the detailed explanations are not repeated here.

The medicine management apparatus 40 further includes: a first authorized user registering unit 41; a first authorized user recording unit 42; a second authorized user registering unit 44; a second authorized user recording unit 45; a management unit 49; an alarm signal generating unit 23; a check signal receiving unit 25; and a check signal recording unit 26.

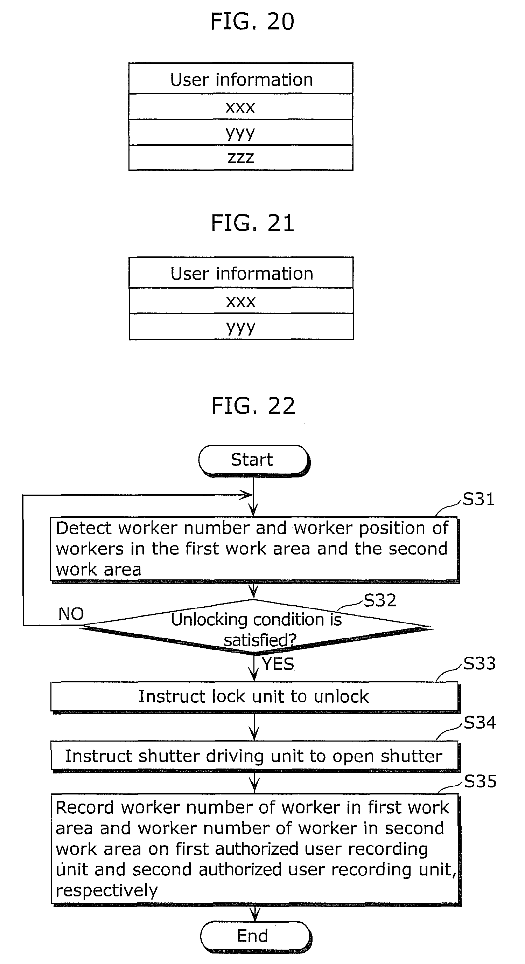

Worker information (worker number) of a worker who is authorized to use the medicine storage 11 is registered, as the first authorized user, on the first authorized user registering unit 41. FIG. 20 is a diagram which shows an example of the worker information of the worker who is authorized to use the medicine storage 11, which is registered on the first authorized user registering unit 41. As shown in FIG. 20, "xxx", "yyy", and "zzz" are recorded as the worker numbers of the workers who are authorized to use the medicine storage 11.

The first authorized user recording unit 42 records the worker information of the first authorized user who uses the medicine storage 11.

A first determining unit 43 determines whether or not the worker information detected by the tag detection unit 14 is registered on the first authorized user recording unit 42.

Worker information of a worker who is authorized to use the checking appratus 50 is registered, as the second authorized user, on the second authorized user registering unit 44. FIG. 21 is a diagram which shows an example of the worker information of the worker who is authorized to use the checking appratus 50, which is registered on the second authorized user registering unit 44. As shown in FIG. 21, "xxx" and "yyy" are registered as the worker numbers of the workers who are authorized to use the checking appratus 50.

The second authorized user recording unit 45 records the worker information of the second authorized user who uses the checking appratus 50.

The second determining unit 46 determines whether or not the worker information detected by the tag detection unit 14 is registered on the second authorized user registering unit 44 or the second authorized user recording unit 45.

The management unit 49 performs a process for managing each of the structural elements included in the medicine management apparatus 40. The management unit 49 includes the first determining unit 43 and the second determining unit 46 described below.

The alarm signal generating unit 23, the check signal receiving unit 25, and the check signal recording unit 26 have the same structures as the structures described in Embodiment 1, and thus the detailed explanations are not repeated here.

According to the medicine management apparatus 40 of Embodiment 3, the lock unit 13 unlocks the shutter 12 of the medicine storage 11 when an unlocking condition is satisfied in the state where the medicine storage 11 is locked. The unlocking condition is a condition that one of the worker information items of two workers detected by the tag detection unit 14 is registered on the first authorized user registering unit 41 and the worker who has the worker information is present in the first work area, and the other is registered on the second authorized user registering unit 44 and the worker who has the worker information is present in the second work area.

In addition, the alarm signal generating unit 23 generates an alarm signal unless the working condition of the medicine management apparatus 10 is satisfied in the state where the shutter 12 of the medicine storage 11 according to Embodiment 3 is unlocked. The unlocking condition of the medicine management apparatus 10 is a condition that one of the worker information items of two workers detected by the tag detection unit 14 is registered on the first authorized user registering unit 41 and the worker who has the worker information is present in the first work area, and the other is registered on the second authorized user registering unit 44 and the worker who has the worker information is present in the second work area.

In addition, the management unit 49 records, on the check signal recording unit 26, the check signal received by the check signal receiving unit 25 when the check condition is satisfied. The check condition is that one of the worker information items of the two workers detected by the tag detection unit 14 is registered on the second authorized user registering unit 44 and the worker who has the worker information is present in the second work area.

It is to be noted that the above-described first authorized user is a medicine dispenser and the second authorized user is a checker. The medicine dispenser is a person who is authorized to perform dispensing of medicines using the medicine management apparatus 40. The checker is a person who is authorized to check the work of the medicine dispenser. The medicine dispenser and the checker are workers, and can be determined by each of the medical institutions.

The following describes a determination process for an unlocking condition carried out at the time of starting dispensing of medicines, with reference to the flow chart shown in FIG. 22.

The tag detection unit 14 detects an ID tag embedded in the name tag 18 of a worker who is present at a detectable position, and transmits, to the management unit 49, a worker number recorded on the ID tag and a detection position of the detected ID tag (S31). At this time, it is possible to simultaneously detect plural ID tags. However, the case where ID tags of two workers are detected is described here.

The first determining unit 43 and the second determining unit 46 of the management unit 49 determines whether or not the worker numbers and the work positions of the two workers transmitted from the tag detection unit 14 satisfy the unlocking condition for unlocking the shutter 12 by the lock unit 13 (S32).

The unlocking condition, here, means that the following two items are simultaneously satisfied.
[Unlocking condition 1] The detected worker number of a worker (one of the workers) is registered on the first authorized user registering unit 41 and the work position is in the first work area 30.
[Unlocking condition 2] The detected worker number of a worker (the other worker) is registered on the second authorized user registering unit 44 and the work position is in the second work area 31.

More specifically, the unlocking condition is that the dispenser is present in the first work area 30 and the checker is present in the second work area 31.

When the two items are not simultaneously satisfied (NO in S32), it is determined that the unlocking condition is not satisfied and the process returns to S31. Then the management unit 49 repeats the determination of whether or not the worker number and the work position transmitted from the tag detection unit 14 satisfy the unlocking condition.

When the unlocking condition is satisfied (YES in S32), the management unit 49 instructs the lock unit 13 to unlock the shutter 12 (S33). The lock unit 13 unlocks the shutter 12 upon receiving the instruction from the management unit 49.

The management unit 49 instructs the shutter driving unit 17 to open the shutter 12 to make the medicine storage 11 available (S34). The shutter driving unit 17, in response to the instruction from the management unit 49, opens the shutter 12 to make the medicine storage 11 available.

The management unit 49 records, on the first authorized user recording unit 42, the worker number of the detected first authorized user (dispenser), and records, on the second authorized user recording unit 45, the worker number of the detected second authorized user (checker) (S35).

As described above, when the shutter 12 is opened and medicines can be taken out from the container 15, it is possible for the dispenser to dispense the medicines.

Next, an operation during the dispensing of medicines using the medicine management apparatus 40 will be described.

The first authorized user and the second authorized user need to be present at the same time in order to perform dispensing using the medicine management apparatus 40. More specifically, the first authorized user needs to be present in the first work area 30 and the second authorized user needs to be present in the second work area 31. According to the medicine management apparatus 40 of Embodiment 3, dispensing of medicines cannot be performed unless the first authorized user and the second authorized user are present at the same time.

Therefore, when dispensing work is performed using the medicine management apparatus 40, the medicine storage 11 does not open unless the first authorized user and the second authorized user are present, and thus double check is performed without fail.

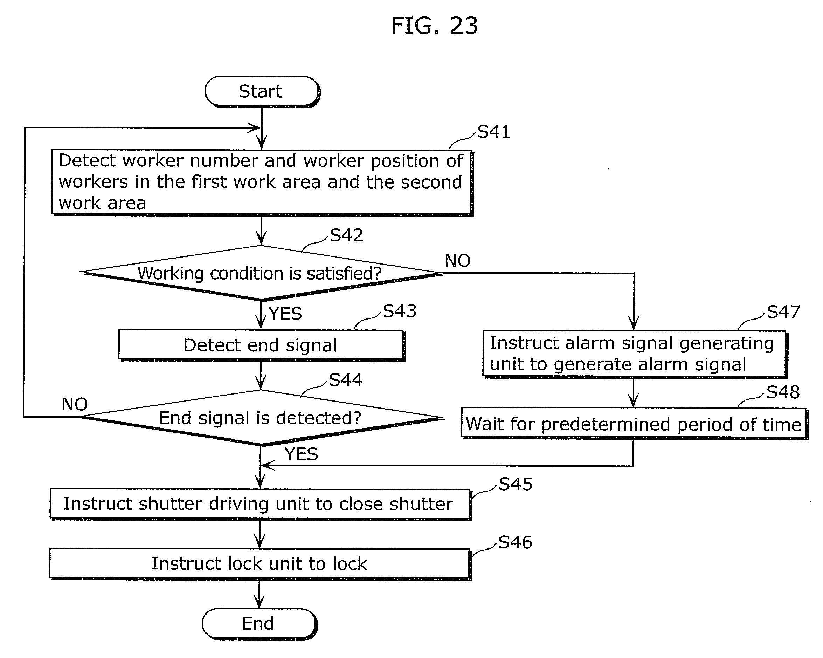

The following describes a determination process for a working condition carried out during the dispensing of medicines, with reference to the flow chart shown in FIG. 23.

The tag detection unit 14 detects an ID tag embedded in the name tag 18 of a worker who is present in a detectable distance, and transmits, to the management unit 49, a worker number recorded on the ID tag and a detection position of the detected ID tag (S41).

At this time, it is possible to simultaneously detect plural ID tags. In this case, the ID tags of two workers are detected and the worker numbers and the work positions of the two workers are transmitted to the management unit 49.

The first determining unit 43 and the second determining unit 46 of the management unit 49 determine whether or not the working condition (a condition for allowing dispensing of medicines) of the medicine management apparatus 40 is satisfied, based on the worker numbers and the work positions of the two workers transmitted from the tag detection unit 14 (S42).

The working condition of the medicine management apparatus 40 described here means that the following two items are simultaneously satisfied.
[Working condition 1] The detected worker number of the first authorized user is recorded on the first authorized user recording unit 42 and the work positions is in the first work area 30.
[Working condition 2] The detected worker number of the second authorized user is recorded on the second authorized user recording unit 45 and the work positions is in the second work area 31.

When the working condition is satisfied (YES in S42), the management unit 49 performs detection of an end signal that is sent from the operation unit 16 when the dispenser presses an end button of the operation unit 16 after the work is completed (S43).

When the end signal of the work is not detected (NO in S44), the process returns to S41 and the management unit 49 repeats the determination of whether or not the working condition of the medicine management apparatus 40 is satisfied.

When the end signal of the work is detected (YES in S44), the management unit 49 instructs the shutter driving unit 17 to close the shutter 12 (S45). Then the shutter 12 is closed to make the medicine storage 11 in a closed state. The management unit 49 instructs the lock unit 13 to lock the shutter 12 (S46).

When the working condition is not satisfied (NO in S42), the management unit 49 instructs the alarm signal generating unit 23 to generate an alarm signal (S47). The alarm signal generating unit 23 generates an alarm signal in response to the instruction. The management unit 49 performs the processes subsequent to S45 after a predetermined waiting period of time (S48).

When the working condition is satisfied as described above, the worker can take out medicines stored in the container 15, and thus it is possible to perform dispensing of medicines. In addition, when the work is to be ended, the worker presses an end button, thereby making the medicine management apparatus 40 in the closed state.

It is to be noted that, when an alarm signal is generated by the alarm signal generating unit 23 in response to the instruction from the management unit 49, the alarm signal is transmitted to an alarm sound generating unit (not illustrated) of the medicine management apparatus 40 to generate an alarm sound from the alarm sound generating unit, thereby notifying, to the surrounding area, that dispensing of medicines is not carried out by two workers.

In addition, when an alarm signal is generated by the alarm signal generating unit 23, the shutter driving unit 17 receives the alarm signal and automatically closes the shutter 12 to make the medicine storage 11 unavailable, and further the lock unit 13 locks the shutter 12, thereby prohibiting carrying out the dispensing of medicines.

Next, an operation during check of medicines using the medicine management apparatus 40 and the checking appratus 50 will be described.

The checker must be a person who is qualified to check medicines. The person who is qualified to check medicines is, for example, a person who is a licensed pharmacist. According to Embodiment 3, a worker whose worker number is registered on the second authorized user registering unit 44 is a licensed pharmacist.

In Embodiment 3, when a check condition that at least one worker who is authorized to check medicines is present in the second work area 31 is satisfied, the medicine management apparatus 40 receives a check result performed by the checking appratus 50. It is to be noted that the check of medicines and dispensing of medicines are performed in parallel.

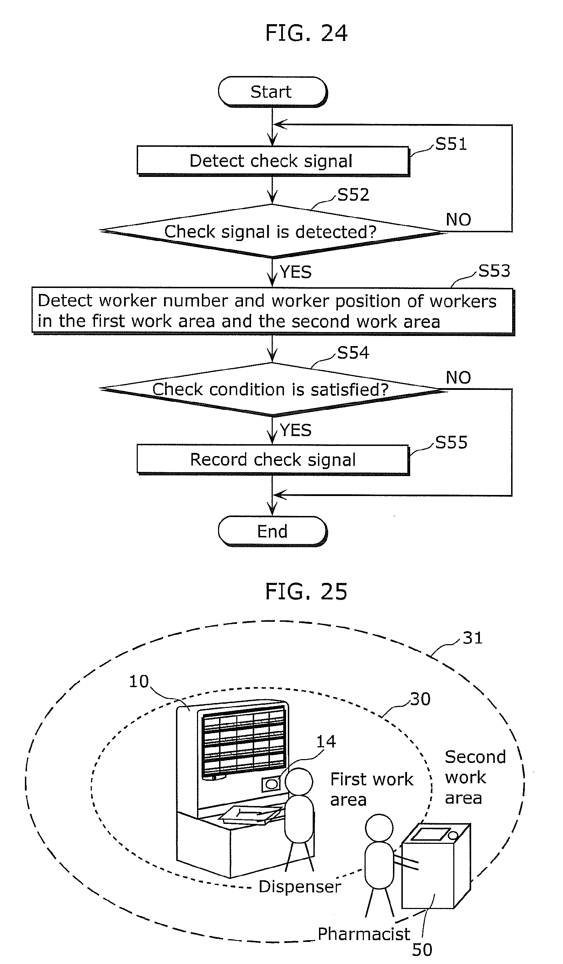

The following describes a determination process for the check condition, which is carried out during the check of medicines, with reference to the flow chart shown in FIG. 24.

In response to an input of a check result by a worker using the check result input unit 51, the control unit 53 transmits check signal indicating the check result to the check signal receiving unit 25.

The management unit 49 detects whether or not the check signal receiving unit 25 receives the check signal (S51). When the check signal is not detected (NO in S52), the process returns to S51.

When the check signal is detected (YES in S52), the tag detection unit 14 detects an ID tag embedded in the name tag 18 of a worker who is present in a detectable distance, and transmits, to the management unit 49, a worker number recorded on the ID tag and a detection position of the detected ID tag (S53).

At this time, it is possible to simultaneously detect plural ID tags. In this case, the ID tags of two workers are detected and the worker numbers and the work positions of the two workers are transmitted to the management unit 49.

The second determining unit 46 of the management unit 49 determines whether or not the check condition of medicines (a condition for allowing check of medicines) is satisfied, from the worker numbers and the work positions of the two workers transmitted from the tag detection unit 14 (S54).

The inspecting condition of medicines, here, means that the following two items are simultaneously satisfied.
[Check condition 1] At least one worker is present in the second work area 31.
[Check condition 2] The worker number of at least one worker present in the second work area 31 is registered on the second authorized user registering unit 44.

Here, an example of the locations of workers when the check condition of medicines is satisfied and not satisfied will be explained.

As shown in FIG. 25, when a person qualified to check medicines (pharmacist) is present in the second work area 31, it is determined that the check condition of medicines is satisfied.

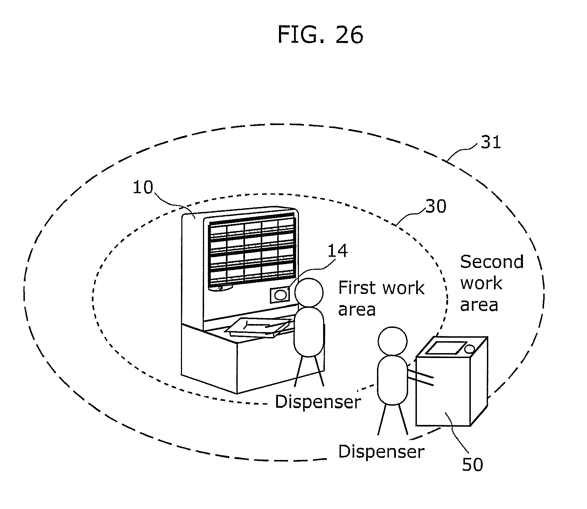

As shown in FIG. 26, when a person qualified to check medicines (pharmacist) is not present in the second work area 31, it is determined that the check condition of medicines is not satisfied. The dispensers shown in FIG. 26 are not qualified as pharmacists.

When the check condition of medicines is satisfied (YES in S54), the management unit 49 records the check signal received by the check signal receiving unit 25, on the check signal recording unit 26 (S55). With this, the result of check performed by the checker is received.

When the check condition of medicines is not satisfied (NO in S54), the management unit 49 ends the process without recording, on the check signal recording unit 26, the check signal received by the check signal receiving unit 25.

As described above, the medicine management apparatus 40 can receive the check result only when the worker whose worker number is registered on the second authorized user registering unit 44 is present in the second work area 31.

It is to be noted that the condition may be set as follows: the checking appratus 50 is located in the second work area 31 and outside the first work area 30 and, instead of the Check condition 1, at least one worker is present in the second work area 31 and outside the first work area 30. This allows clear separation between the position of the medicine management apparatus 40 and the position of the checking appratus 50, and thus it is possible to accurately identify the worker number of the worker who operates the checking appratus 50.

As described above, with the medicine management apparatus 40 according to Embodiment 3, it is possible to provide a medicine management apparatus that allows double check reliably performed by two workers even when the roles of the two workers for performing the double check are clearly separated.

It is to be noted that the medicine management apparatus 40 identifies the worker and detects the work position using the tag detection unit 14; however, a camera and an image analyzing unit may be provided instead of the tag detection unit 14 to identify the worker and detect the work position using an image captured by the camera as in Embodiment 2.

The medicine management apparatus and the checking appratus according to embodiments of the present invention have been described above; however, the present invention is not limited to these embodiments.

The number of the workers is two in Embodiments 1 to 3; however, the number is not limited to this. It is possible to perform processes according to the above-described unlocking condition, working condition, and check condition even when there are three or more workers.

In addition, the medicine management apparatus for performing dispensing of medicines and the checking appratus for checking the dispensed medicine are provided as different bodies in Embodiments 1 to 3; however, the medicine management apparatus may have the function of the checking appratus. Modifications 1 and 2 below will describe this in detail.

### (Modification 1)

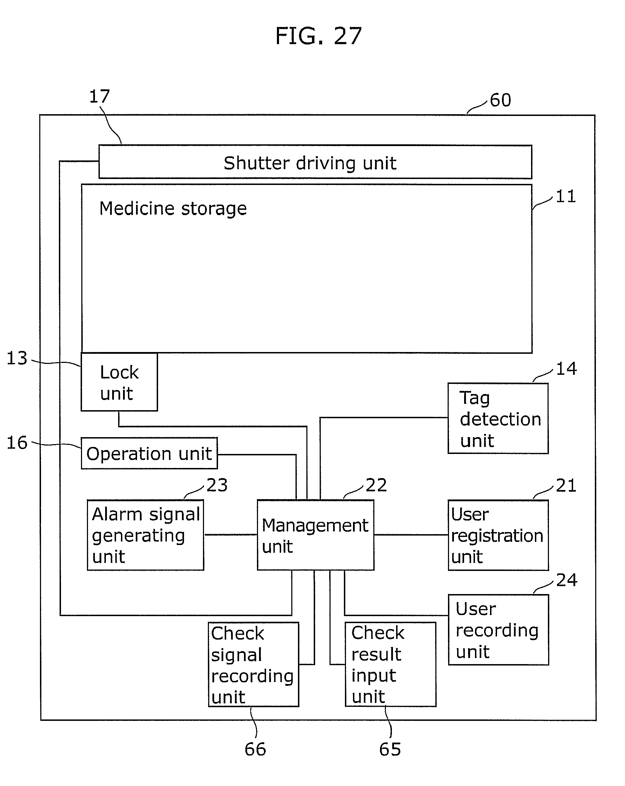

FIG. 27 is a block diagram which shows a functional structure of a medicine management apparatus of which the function of the checking appratus is provided to the medicine management apparatus 10 according to Embodiment 1.

A medicine management apparatus 60 includes a check result input unit 65 and a check signal recording unit 66 instead of the check signal receiving unit 25 and the check signal recording unit 26 in the structure of the medicine management apparatus 10 according to Embodiment 1.

The check result input unit 65 is an interface to input a check result by a checker, and formed as a button or the like.

The check signal recording unit 66 records the check signal indicating the check result inputted by the check result input unit 65.

With the above-described structure, processes same as the processes of Embodiment 1 are performed. The unlocking condition and the working condition of the medicine management apparatus 60 are the same as those in Embodiment 1.

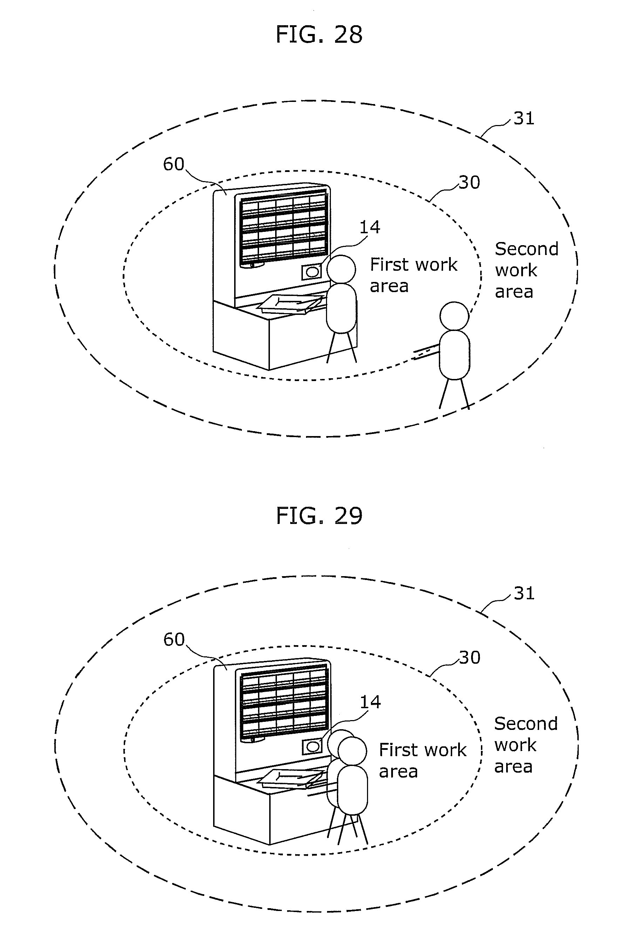

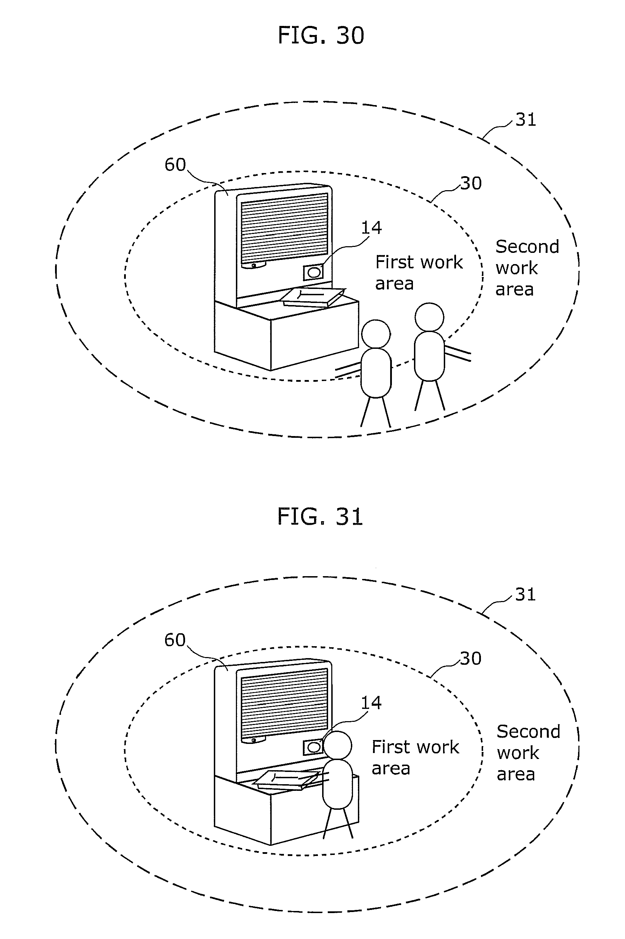

More specifically, in the locked state, the unlocking condition is satisfied in the state shown in FIG. 28 and FIG. 29, and the unlocking condition is not satisfied in the state shown in FIG. 30 and FIG. 31.

More specifically, when a worker is present in the first work area 30 and a worker is present in the second work area 31 as shown in FIG. 28, it is determined that the unlocking condition is satisfied. Here, the worker is a person whose worker number is registered on the user registration unit 21.

In addition, as shown in FIG. 29, when two workers are present in the first work area 30, it is also determined that the unlocking condition is satisfied. Here, the two workers are people whose worker numbers are registered on the user registration unit 21.

In addition, as shown in FIG. 30, when two workers are present in the second work area 31 and outside the first work area 30, it is determined that the unlocking condition is not satisfied. Here, the two workers are people whose worker numbers are registered on the user registration unit 21.

In addition, as shown in FIG. 31, when one worker is present in the first work area 30 but no worker is present in the second work area 31, it is determined that the unlocking condition is not satisfied. Here, the worker is a person whose worker number is registered on the user registration unit 21.

Likewise, the working condition of the medicine management apparatus 60 is satisfied in the states shown in FIG. 28 and FIG. 29, and not satisfied in the states shown in FIG. 30 and FIG. 31.

The check condition of medicines is different from that in Embodiment 1. More specifically, the checking appratus 50 is located in the second area in Embodiment 1; however, the medicine management apparatus 60 located in the first area is used for performing the check of medicines in Modification 1. Accordingly, the check condition of medicines in Modification 1 means that the following two items are simultaneously satisfied.
[Check condition 1'] At least one worker is present in the first work area 30.
[Check condition 2'] The worker number of at least one worker present in the first work area 30 is registered on the user registration unit 21.

The management unit 22 records, on the check signal recording unit 66, a check signal indicating a check result inputted by the check result input unit 65 when the check condition is satisfied, and deletes a check signal indicating a check result inputted by the check result input unit 65 and do not record on the check signal recording unit 66 when the check condition is not satisfied.

### (Modification 2)

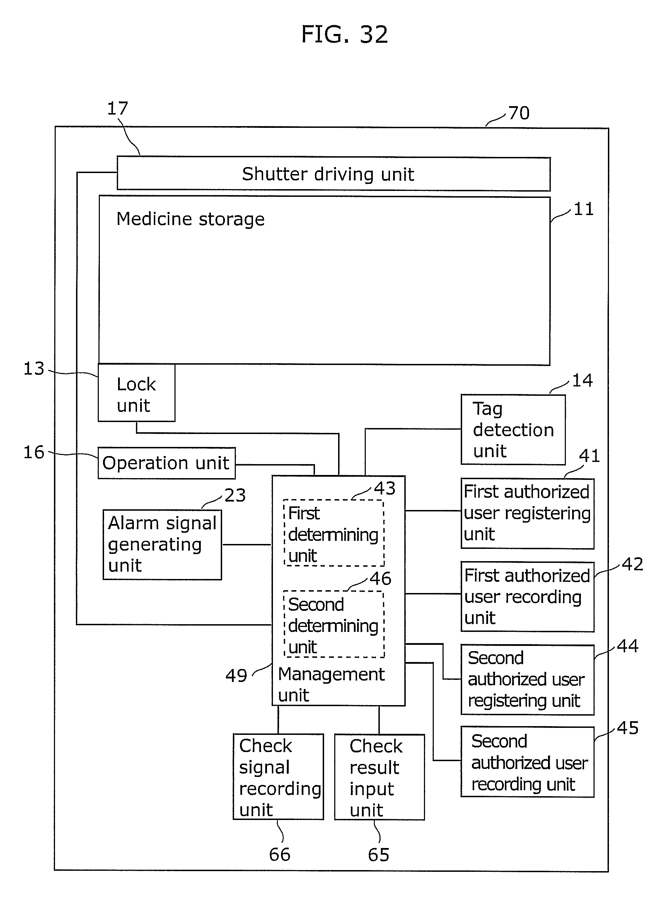

FIG. 32 is a block diagram which shows a functional structure of a medicine management apparatus of which the function of the checking appratus is provided to the medicine management apparatus 40 according to Embodiment 3.

A medicine management apparatus 70 includes a check result input unit 65 and a check signal recording unit 66 instead of the check signal receiving unit 25 and the check signal recording unit 26 in the structure of the medicine management apparatus 40 according to Embodiment 3.

The check result input unit 65 and the check signal recording unit 66 are the same as those described in Modification 1, and thus the detailed explanation for them is not repeated.

With the above-described structure, processes same as the processes of Embodiment 3 are performed. The unlocking condition and the working condition of the medicine management apparatus 70 are the same as those in Embodiment 3.

The check condition of medicines is different from that in Embodiment 3. More specifically, the checking appratus 50 is located in the second area in Embodiment 3; however, the medicine management apparatus 70 located in the first area is used for performing the check of medicines in Modification 2. Accordingly, the check condition of medicines in Modification 2 means that the following two items are simultaneously satisfied.
[Check condition 1"] At least one worker is present in the first work area 30.
[Check condition 2"] The worker number of at least one worker present in the first work area 30 is registered on the second authorized user registering unit 44.

The management unit 49 records, on the check signal recording unit 66, a check signal indicating a check result inputted by the check result input unit 65 when the check condition is satisfied, and deletes a check signal indicating a check result inputted by the check result input unit 65 and do not record on the check signal recording unit 66 when the check condition is not satisfied.

Here, an example of the locations of workers when the check condition of medicines is satisfied and not satisfied will be explained.

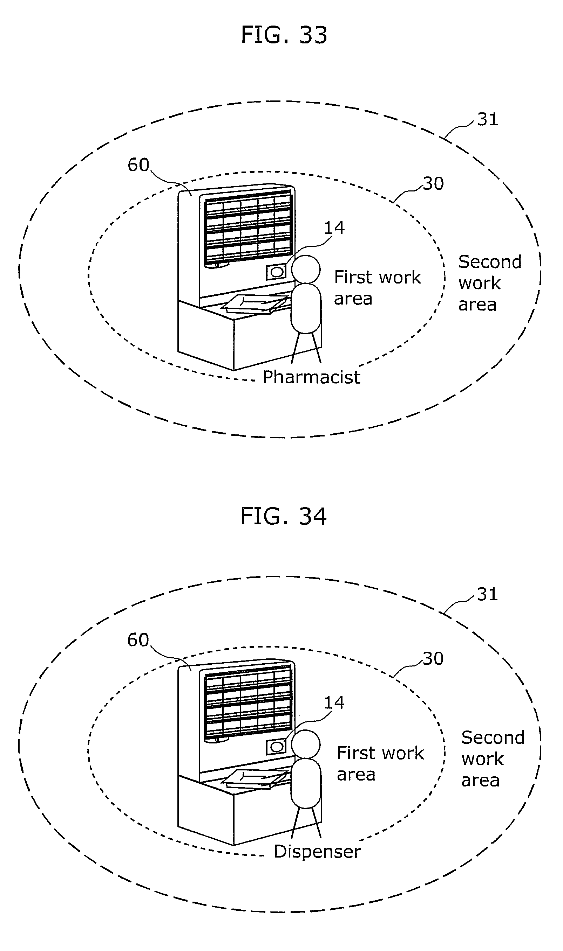

As shown in FIG. 33, when a person qualified to check medicines (pharmacist) is present in the first work area 30, it is determined that the check condition of medicines is satisfied.

As shown in FIG. 34, when a person qualified to check medicines (pharmacist) is not present in the first work area 30, it is determined that the check condition of medicines is not satisfied. The dispenser shown in FIG. 34 is not qualified as a pharmacist.

In addition, each of the aforementioned apparatuses may be, specifically, a computer system including a microprocessor, a ROM, a RAM, a hard disk drive, a display unit, a keyboard, a mouse, and so on. A computer program is stored in the RAM or hard disk drive. The respective apparatuses achieve their functions through the microprocessor's operation according to the computer program. Here, the computer program is configured by combining plural instruction codes indicating instructions for the computer in order to achieve predetermined functions.

In addition, a part or all of the constituent elements constituting the respective apparatuses may be configured from a single System-LSI (Large-Scale Integration). The System-LSI is a super-multi-function LSI manufactured by integrating constituent units on one chip, and is specifically a computer system configured by including a microprocessor, a ROM, a RAM, and so on. A computer program is stored in the RAM. The System-LSI achieves its function through the microprocessor's operation according to the computer program.

In addition, a part or all of the constituent elements constituting the respective apparatuses may be configured as an IC card which can be attached and detached from the respective apparatuses or as a stand-alone module. The IC card or the module is a computer system configured from a microprocessor, a ROM, a RAM, and so on. The IC card or the module may also be included in the aforementioned super-multi-function LSI. The IC card or the module achieves its function through the microprocessor's operation according to the computer program. The IC card or the module may also be implemented to be tamper-resistant.

In addition, the present invention may also be realized as a method described above. In addition, the present invention, may be a computer program for realizing the previously illustrated method, using a computer, and may also be a digital signal including the computer program.

Furthermore, the present invention may also be realized by storing the computer program or the digital signal in a non-transitory computer readable recording medium such as flexible disc, a hard disk, a CD-ROM, an MO, a DVD, a DVD-ROM, a DVD-RAM, a BD (Blu-ray Disc®), and a semiconductor memory. Furthermore, the present invention may also include the digital signal recorded in these non-transitory recording media.

Furthermore, the present invention may also be realized by the transmission of the aforementioned computer program or digital signal via a telecommunication line, a wireless or wired communication line, a network represented by the Internet, a data broadcast and so on.

The present invention may also be a computer system including a microprocessor and a memory, in which the memory stores the aforementioned computer program and the microprocessor operates according to the computer program.

Furthermore, by transferring the program or the digital signal by recording onto the aforementioned non-transitory recording media, or by transferring the program or digital signal via the aforementioned network and the like, execution using another independent computer system is also made possible.

In addition, each of the above-mentioned embodiments and modifications may be combined with each other.

It is to be noted that the essential structural elements of the medicine management apparatus according to the present invention are the medicine storage 11, the lock unit 13, the tag detection unit 14, and the management unit 22, and it is desirable that other structural elements are included in the medicine management apparatus.

It is to be understood that the embodiments disclosed above are intended not for the purpose of limitation but for exemplification only in all respects. The scope of this invention is indicated not by the aforementioned description but by the Claims, and it is intended that various changes and modifications within the same or equivalent meaning and scope of the Claims may be included.

### [Industrial Applicability]

The present invention can be applied to a method of controlling the medicine management apparatus for managing medicine and a medicine management apparatus and so on, and is especially useful in dispensing of medicines which requires double check for performing the dispensing of medicines.

### [Reference Signs List]

- 10, 33, 40, 60, 70: medicine management apparatus
- 11: medicine storage
- 12: shutter
- 13: lock unit
- 14: tag detection unit
- 15: container
- 16: operation unit
- 17: shutter driving unit
- 18: name tag
- 19: IC
- 20: antenna
- 21: user registration unit
- 22, 49: management unit
- 23: alarm signal generating unit
- 24: user recording unit
- 25: check signal receiving unit
- 26, 66: check signal recording unit
- 30: first work area
- 31: second work area
- 32: camera
- 39: buffer area
- 41: first authorized user registering unit
- 42: first authorized user recording unit
- 43: first determining unit
- 44: second authorized user registering unit
- 45: second authorized user recording unit
- 46: second determining unit
- 50: checking appratus
- 51: check result input unit
- 52: display unit
- 53: control unit
- 65: check result input unit

## Claims

1. A method of controlling a medicine management apparatus that includes a medicine storage and a detection unit that detects worker information for identifying a worker and a work position of the worker,
wherein a first work area and a second work area are preliminarily specified, the first work area including an installation position of the medicine management apparatus, and the second work area including a position from which the worker who operates the medicine management apparatus is directly visible,
the method of controlling a medicine management apparatus comprising:
detecting the worker information and the work position of the worker;
determining whether or not an unlocking condition is satisfied, based on a result of the detecting, the unlocking condition being that, in a state where the medicine storage is locked, (i) a plurality of workers authorized to use the medicine storage are present in an area that is a combination of the first work area and the second work area and (ii) at least one of the workers is present in the first work area; and
setting the medicine storage to a state which allows the medicine storage to be unlocked, when it is determined, in the determining whether or not an unlocking condition is satisfied, that the unlocking condition is satisfied.

2. The method of controlling a medicine management apparatus according to Claim 1,
the method further comprising:
determining whether or not a working condition is satisfied, based on the result of the detecting, the working condition being that, in a state where the medicine storage is unlocked, (i) the plurality of workers authorized to use the medicine storage are present in the area that is the combination of the first work area and the second work area and (ii) the at least one of the workers is present in the first work area; and
generating an alert signal when it is determined, in the determining whether or not a working condition is satisfied, that the working condition is not satisfied.

3. The method of controlling a medicine management apparatus according to Claim 2, the method further comprising
locking the medicine storage when it is determined, in the determining whether or not a working condition is satisfied, that the working condition is not satisfied.

4. The method of controlling a medicine management apparatus according to any one of Claims 1 to 3,
wherein in the detecting, the worker information and the work position of the worker are detected by reading the worker information in a non-contact manner, the worker information being stored in an ID tag held by the worker.

5. The method of controlling a medicine management apparatus according to any one of Claims 1 to 3,
wherein in the detecting, the worker information and the work position of the worker are detected by analyzing a captured image of the worker.

6. The method of controlling a medicine management apparatus according to any one of Claims 1 to 5,
wherein the medicine management apparatus further includes a user registration unit in which user information of a worker authorized to use the medicine management apparatus is stored, and
in the determining whether or not an unlocking condition is satisfied, whether or not the worker detected in the detecting is one of the workers authorized to use the medicine storage is determined by determining whether or not the worker information of the worker detected in the detecting is stored in the user registration unit.

7. The method of controlling a medicine management apparatus according to one of Claims 2 and 3,
wherein the medicine management apparatus further includes a user registration unit in which user information of a worker authorized to use the medicine management apparatus is stored, and
in the determining whether or not a working condition is satisfied , whether or not the worker detected in the detecting is one of the workers authorized to use the medicine storage is determined by determining whether or not the worker information of the worker detected in the detecting is stored in the user registration unit.

8. The method of controlling a medicine management apparatus according to any one of Claims 1 to 7,
wherein in the determining whether or not an unlocking condition is satisfied, whether or not the unlocking condition is satisfied is determined based on a result of the detection in the detecting, the unlocking condition being that, in the state where the medicine storage is locked, (i) at least one of the workers authorized to work in the first work area is present in the first work area and (ii) at least one of the workers authorized to work in the second work area is present in the second work area.

9. The method of controlling a medicine management apparatus according to Claim 8,
wherein the medicine management apparatus further includes (i) a first authorized user registering unit in which use information of the worker authorized to work in the first work area is stored and (ii) a second authorized user registering unit in which use information of the worker authorized to work in the second work area is stored, and
the determining whether or not an unlocking condition is satisfied includes:
determining whether or not worker information of a worker present in the first work area is stored in the first authorized user registering unit, to determine whether or not the worker present in the first work area is the worker authorized to work in the first work area, the worker information being detected in the detecting; and
determining whether or not worker information of a worker present in the second work area is stored in the second authorized user registering unit, to determine whether or not the worker present in the second work area is the worker authorized to work in the second work area, the worker information being detected in the detecting.

10. The method of controlling a medicine management apparatus according to one of Claims 2 and 3,
wherein in the determining whether or not a working condition is satisfied, whether or not the working condition is satisfied is determined, based on a result of the detection in the detecting, the working condition being that, in the state where the medicine storage is unlocked, (i) at least one of the workers authorized to work in the first work area is present in the first work area and (ii) at least one of the workers authorized to work in the second work area is present in the second work area.

11. The method of controlling a medicine management apparatus according to Claim 10,
wherein the medicine management apparatus further includes (i) a first authorized user registering unit in which use information of a worker authorized to work in the first work area is stored and (ii) a second authorized user registering unit in which use information of a worker authorized to work in the second work area is stored, and
the determining whether or not a working condition is satisfied includes:
determining whether or not worker information of the worker present in the first work area is stored in the first authorized user registering unit, to determine whether or not the worker present in the first work area is the worker authorized to work in the first work area, the worker information being detected in the detecting; and
determining whether or not worker information of the worker present in the second work area is stored in the second authorized user registering unit, to determine whether or not the worker present in the second work area is the worker authorized to work in the second work area, the worker information being detected in the detecting.

12. The method of controlling a medicine management apparatus according to any one of Claims 1 to 11,
wherein a checking appratus for receiving a result of check performed by a worker is located in the second work area, and
the method further comprises:
determining whether or not a check condition is satisfied, the check condition being that at least one checker who is a worker authorized to check medicines is present in the second work area; and
receiving a result of check performed by the checker when it is determined, in the determining whether or not a check condition is satisfied, that the check condition is satisfied.

13. The method of controlling a medicine management apparatus according to Claim 12,
wherein the checking appratus is located in the second work area and outside the first work area, and
in the determining whether or not a check condition is satisfied, it is determined whether or not a check condition that the at least one checker is present in the second work area and outside the first work area is satisfied.

14. The method of controlling a medicine management apparatus according to Claim 13,
wherein the first work area and the second work area are preliminarily specified such that a worker cannot operate the checking appratus from the first work area and a worker cannot operate the medicine management apparatus from the second work area.

15. The method of controlling a medicine management apparatus according to Claim 14,
wherein an area which belongs to none of the work areas is provided between the first work area and the second work area.

16. The method of controlling a medicine management apparatus according to any one of Claims 1 to 11,
wherein the medicine management apparatus further includes a check result receiving unit for receiving a result of check, and
the method further comprises:
determining whether or not a check condition is satisfied, the check condition being that at least one checker who is a worker authorized to check medicines is present in the first work area; and
receiving a result of check performed by the checker when it is determined, in the determining whether or not a check condition is satisfied, that the check condition is satisfied.

17. The method of controlling a medicine management apparatus according to Claim 16,
wherein in the determining whether or not a check condition is satisfied, it is determined whether or not a check condition that the at least one checker is present in the first work area and a worker unauthorized to check medicines is not present in the first work area is satisfied.

18. A medicine management apparatus which manages medicines, the appratus comprising:
a medicine storage;
a detection unit configured to detect worker information for identifying a worker and a work position of the worker;
a management unit configured to determine whether or not an unlocking condition is satisfied, based on a result of detection performed by the detection unit, in a state where (i) a first work area including an installation position of the medicine management apparatus and a second work area including a position from which the worker who operates the medicine management apparatus is directly visible are preliminarily specified and (ii) the medicine storage is locked, the unlocking condition being that a plurality of workers authorized to use the medicine storage are present in an area that is a combination of the first work area and the second work area and at least one of the workers is present in the first work area; and
a lock unit configured to set the medicine storage to a state which allows the medicine storage to be unlocked, when it is determined, by the management unit, that the unlocking condition is satisfied.

19. A program for controlling a medicine management apparatus including a medicine storage and a detection unit that detects worker information for identifying a worker and a work position of the worker, the program causing a computer to execute:
detecting the worker information and the work position of the worker;
determining whether or not an unlocking condition is satisfied, based on a result of the detecting, the unlocking condition being that, in a state where a first work area and a second work area are preliminarily specified and the medicine storage is locked, (i) a plurality of workers authorized to use the medicine storage are present in an area that is a combination of the first work area and the second work area and (ii) at least one of the workers is present in the first work area, the first work area including an installation position of the medicine management apparatus, and the second work area including a position from which the worker who operates the medicine management apparatus is directly visible; and
setting the medicine storage to a state which allows the medicine storage to be unlocked, when it is determined, in the determining whether or not an unlocking condition is satisfied, that the unlocking condition is satisfied.
